# EUROPÄISCHE PATENTSCHRIFT

(11) **EP 2 945 645 B1**
(45) Veröffentlichungstag und Bekanntmachung des Hinweises auf die Patenterteilung: **18.09.2019**
(21) Anmeldenummer: 14705286.4
(22) Anmeldetag: 17.01.2014
(51) Int. Cl.: A61K 38/48, A61K 48/00, A61P 35/00

(54) **SELEKTIVES ZELLTOD-INDUZIERENDES BINÄRES ENZYMSYSTEM**
SELECTIVE CELL-DEATH-INDUCING BINARY ENZYME SYSTEM
SYSTÈME ENZYMATIQUE BINAIRE SÉLECTIF INDUISANT L'APOPTOSE

(30) Priorität: 17.01.2013 EP 13151753
(43) Veröffentlichungstag der Anmeldung: 25.11.2015
(73) Patentinhaber: SIT Soft Intelligent Therapeutics GmbH & Co KG, 44227 Dortmund (DE)
(72) Erfinder: TOUREL, Sylvain, 45525 Hattingen (DE)
(74) Vertreter: Simandi, Claus
(86) Internationale Anmeldenummer: PCT/EP2014/050966
(87) Internationale Veröffentlichungsnummer: WO 2014/111553

(56) Entgegenhaltungen:
- EP-A1- 1 873 251
- WO-A2-03/007889
- NALLAMSETTY S ET AL: "Efficient site-specific processing of fusion proteins by tobacco vein mottling virus protease in vivo and in vitro", PROTEIN EXPRESSION AND PURIFICATION, ACADEMIC PRESS, SAN DIEGO, CA, Bd. 38, Nr. 1, 1. November 2004 (2004-11-01), Seiten 108-115, XP004599054, ISSN: 1046-5928, DOI: 10.1016/J.PEP.2004.08.016
- KREPELA: "Granzyme B-induced apoptosis in cancer cells and its regulation (Review)", INTERNATIONAL JOURNAL OF ONCOLOGY, Bd. 37, Nr. 6, 22. Oktober 2010 (2010-10-22), XP055108777, ISSN: 1019-6439, DOI: 10.3892/ijo_00000788

### SEQUENCE LISTING

<110> SIT Soft Intelligent Therapeutics GmbH & Co KG
<120> Selektives Zelltod-induzierendes binäres Enzymsystem
<130> SIT126-03WO
<150> EP 13 151 753,4
   <151> 2013-01-17
<160> 9
<170> PatentIn version 3.3
<210> 1
   <211> 247
   <212> PRT
   <213> Homo sapiens
<400> 1
   Met Gln Pro Ile Leu Leu Leu Leu Ala Phe Leu Leu Leu Pro Arg Ala
   1               5                   10                  15
   Asp Ala Gly Glu Ile Ile Gly Gly His Glu Ala Lys Pro His Ser Arg
               20                  25                  30
   Pro Tyr Met Ala Tyr Leu Met Ile Trp Asp Gln Lys Ser Leu Lys Arg
           35                  40                  45
   Cys Gly Gly Phe Leu Ile Arg Asp Asp Phe Val Leu Thr Ala Ala His
       50                  55                  60
   Cys Trp Gly Ser Ser Ile Asn Val Thr Leu Gly Ala His Asn Ile Lys
   65                  70                  75                  80
   Glu Gln Glu Pro Thr Gln Gln Phe Ile Pro Val Lys Arg Pro Ile Pro
                   85                  90                  95
   His Pro Ala Tyr Asn Pro Lys Asn Phe Ser Asn Asp Ile Met Leu Leu
               100                 105                 110
   Gln Leu Glu Arg Lys Ala Lys Arg Thr Arg Ala Val Gln Pro Leu Arg
           115                 120                 125
   Leu Pro Ser Asn Lys Ala Gln Val Lys Pro Gly Gln Thr Cys Ser Val
       130                 135                 140
   Ala Gly Trp Gly Gln Thr Ala Pro Leu Gly Lys His Ser His Thr Leu
   145                 150                 155                 160
   Gln Glu Val Lys Met Thr Val Gln Glu Asp Arg Lys Cys Glu Ser Asp
                   165                 170                 175
   Leu Arg His Tyr Tyr Asp Ser Thr Ile Glu Leu Cys Val Gly Asp Pro
               180                 185                 190
   Glu Ile Lys Lys Thr Ser Phe Lys Gly Asp Ser Gly Gly Pro Leu Val
           195                 200                 205
   Cys Asn Lys Val Ala Gln Gly Ile Val Ser Tyr Gly Arg Asn Asn Gly
       210                 215                 220
   Met Pro Pro Arg Ala Cys Thr Lys Val Ser Ser Phe Val His Trp Ile
   225                 230                 235                 240
   Lys Lys Thr Met Lys Arg Tyr
                   245
<210> 2
   <211> 229
   <212> PRT
   <213> Homo sapiens
<400> 2
   Ile Ile Gly Gly His Glu Ala Lys Pro His Ser Arg Pro Tyr Met Ala
   1               5                   10                  15
   Tyr Leu Met Ile Trp Asp Gln Lys Ser Leu Lys Arg Cys Gly Gly Phe
               20                  25                  30
   Leu Ile Arg Asp Asp Phe Val Leu Thr Ala Ala His Cys Trp Gly Ser
           35                  40                  45
   Ser Ile Asn Val Thr Leu Gly Ala His Asn Ile Lys Glu Gln Glu Pro
       50                  55                  60
   Thr Gln Gln Phe Ile Pro Val Lys Arg Pro Ile Pro His Pro Ala Tyr
   65                  70                  75                  80
   Asn Pro Lys Asn Phe Ser Asn Asp Ile Met Leu Leu Gln Leu Glu Arg
                   85                  90                  95
   Lys Ala Lys Arg Thr Arg Ala Val Gln Pro Leu Arg Leu Pro Ser Asn
               100                 105                 110
   Lys Ala Gln Val Lys Pro Gly Gln Thr Cys Ser Val Ala Gly Trp Gly
           115                 120                 125
   Gln Thr Ala Pro Leu Gly Lys His Ser His Thr Leu Gln Glu Val Lys
       130                 135                 140
   Met Thr Val Gln Glu Asp Arg Lys Cys Glu Ser Asp Leu Arg His Tyr
   145                 150                 155                 160
   Tyr Asp Ser Thr Ile Glu Leu Cys Val Gly Asp Pro Glu Ile Lys Lys
                   165                 170                 175
   Thr Ser Phe Lys Gly Asp Ser Gly Gly Pro Leu Val Cys Asn Lys Val
               180                 185                 190
   Ala Gln Gly Ile Val Ser Tyr Gly Arg Asn Asn Gly Met Pro Pro Arg
           195                 200                 205
   Ala Cys Thr Lys Val Ser Ser Phe Val His Trp Ile Lys Lys Thr Met
       210                 215                 220
   Lys Arg Tyr Gly Thr
   225
<210> 3
   <211> 260
   <212> PRT
   <213> artificial
<220>
   <223> Insert
<400> 3
   Met Asp Tyr Lys Asp Asp Asp Asp Lys Gly Asp Tyr Lys Asp Asp Asp
   1               5                   10                  15
   Asp Lys Gly Gly Gly Thr Glu Leu Gly Glu Asn Leu Tyr Phe Gln Ile
               20                  25                  30
   Ile Gly Gly His Glu Ala Lys Pro His Ser Arg Pro Tyr Met Ala Tyr
           35                  40                  45
   Leu Met Ile Trp Asp Gln Lys Ser Leu Lys Arg Cys Gly Gly Phe Leu
       50                  55                  60
   Ile Arg Asp Asp Phe Val Leu Thr Ala Ala His Cys Trp Gly Ser Ser
   65                  70                  75                  80
   Ile Asn Val Thr Leu Gly Ala His Asn Ile Lys Glu Gln Glu Pro Thr
                   85                  90                  95
   Gln Gln Phe Ile Pro Val Lys Arg Ala Ile Pro His Pro Ala Tyr Asn
               100                 105                 110
   Pro Lys Asn Phe Ser Asn Asp Ile Met Leu Leu Gln Leu Glu Arg Lys
   115 120 125
   Ala Lys Arg Thr Arg Ala Val Gln Pro Leu Arg Leu Pro Ser Asn Lys
   130 135 140
   Ala Gln Val Lys Pro Gly Gln Thr Cys Ser Val Ala Gly Trp Gly Gln
   145 150 155 160
   Thr Ala Pro Leu Gly Lys His Ser His Thr Leu Gln Glu Val Lys Met
   165 170 175
   Thr Val Gln Glu Asp Arg Lys Cys Glu Ser Asp Leu Arg His Tyr Tyr
   180 185 190
   Asp Ser Thr Ile Glu Leu Cys Val Gly Asp Pro Glu Ile Lys Lys Thr
   195 200 205
   Ser Phe Lys Gly Asp Ser Gly Gly Pro Leu Val Cys Asn Lys Val Ala
   210 215 220
   Gln Gly Ile Val Ser Tyr Gly Arg Asn Asn Gly Met Pro Pro Arg Ala
   225 230 235 240
   Cys Thr Lys Val Ser Ser Phe Val His Trp Ile Lys Lys Thr Met Lys
   245 250 255
   Arg Tyr Gly Thr
   260
<210> 4
   <211> 243
   <212> PRT
   <213> tobacco etch virus
<400> 4
   Met Gly Glu Ser Leu Phe Lys Gly Pro Arg Asp Tyr Asn Pro Ile Ser
   1 5 10 15
   Ser Thr Ile Cys His Leu Thr Asn Glu Ser Asp Gly His Thr Thr Ser
   20 25 30
   Leu Tyr Gly Ile Gly Phe Gly Pro Phe Ile Ile Thr Asn Lys His Leu
   35 40 45
   Phe Arg Arg Asn Asn Gly Thr Leu Leu Val Gln Ser Leu His Gly Val
   50 55 60
   Phe Lys Val Lys Asp Thr Thr Thr Leu Gln Gln His Leu Ile Asp Gly
   65                  70                  75                  80
   Arg Asp Met Met Ile Ile Arg Met Pro Lys Asp Phe Pro Pro Phe Pro
                   85                  90                  95
   Gln Lys Leu Lys Phe Arg Glu Pro Gln Arg Glu Glu Arg Ile Cys Leu
               100                 105                 110
   Val Thr Thr Asn Phe Gln Ala Lys Ser Met Ser Ser Met Val Ser Asp
           115                 120                 125
   Thr Ser Cys Thr Phe Pro Ser Ser Asp Gly Ile Phe Trp Lys His Trp
       130                 135                 140
   Ile Gln Thr Lys Asp Gly Gln Cys Gly Ser Pro Leu Val Ser Thr Arg
   145                 150                 155                 160
   Asp Gly Phe Ile Val Gly Ile His Ser Ala Ser Asn Phe Thr Asn Thr
                   165                 170                 175
   Asn Asn Tyr Phe Thr Ser Val Pro Lys Asn Phe Met Glu Leu Leu Thr
               180                 185                 190
   Asn Gln Glu Ala Gln Gln Trp Val Ser Gly Trp Arg Leu Asn Ala Asp
           195                 200                 205
   Ser Val Leu Trp Gly Gly His Lys Val Phe Met Val Lys Pro Glu Glu
       210                 215                 220
   Pro Phe Gln Pro Val Lys Glu Ala Thr Gln Leu Met Asn Glu Leu Val
   225                 230                 235                 240
   Tyr Ser Gln
<210> 5
   <211> 262
   <212> PRT
   <213> tobacco etch virus
<400> 5
   Met Lys His His His His His His Pro Met Ser Gly Leu Val Pro Arg
   1               5                   10                  15
   Gly Ser Ala Met Gly Glu Ser Leu Phe Lys Gly Pro Arg Asp Tyr Asn
               20                  25                  30
   Pro Ile Ser Ser Thr Ile Cys His Leu Thr Asn Glu Ser Asp Gly His
           35                  40                  45
   Thr Thr Ser Leu Tyr Gly Ile Gly Phe Gly Pro Phe Ile Ile Thr Asn
       50                  55                  60
   Lys His Leu Phe Arg Arg Asn Asn Gly Thr Leu Leu Val Gln Ser Leu
   65                  70                  75                  80
   His Gly Val Phe Lys Val Lys Asp Thr Thr Thr Leu Gln Gln His Leu
                   85                  90                  95
   Ile Asp Gly Arg Asp Met Met Ile Ile Arg Met Pro Lys Asp Phe Pro
               100                 105                 110
   Pro Phe Pro Gln Lys Leu Lys Phe Arg Glu Pro Gln Arg Glu Glu Arg
           115                 120                 125
   Ile Cys Leu Val Thr Thr Asn Phe Gln Ala Lys Ser Met Ser Ser Met
       130                 135                 140
   Val Ser Asp Thr Ser Cys Thr Phe Pro Ser Ser Asp Gly Ile Phe Trp
   145                 150                 155                 160
   Lys His Trp Ile Gln Thr Lys Asp Gly Gln Cys Gly Ser Pro Leu Val
                   165                 170                 175
   Ser Thr Arg Asp Gly Phe Ile Val Gly Ile His Ser Ala Ser Asn Phe
               180                 185                 190
   Thr Asn Thr Asn Asn Tyr Phe Thr Ser Val Pro Lys Asn Phe Met Glu
           195                 200                 205
   Leu Leu Thr Asn Gln Glu Ala Gln Gln Trp Val Ser Gly Trp Arg Leu
       210                 215                 220
   Asn Ala Asp Ser Val Leu Trp Gly Gly His Lys Val Phe Met Val Lys
   225                 230                 235                 240
   Pro Glu Glu Pro Phe Gln Pro Val Lys Glu Ala Thr Gln Leu Met Asn
                   245                 250                 255
   Glu Leu Val Tyr Ser Gln
               260
<210> 6
   <211> 6
   <212> PRT
   <213> Recognition site for TEV
<400> 6
   Glu Asn Leu Tyr Phe Gln
   1 5
<210> 7
   <211> 7
   <212> PRT
   <213> Recognition site for TEV
<400> 7
   Glu Asn Leu Tyr Phe Gln Gly
   1 5
<210> 8
   <211> 774
   <212> DNA
   <213> artificial
<220>
   <223> Coding for SEQ ID No. 3
<220>
   <221> misc_feature
   <222> (5)..(5)
   <223> n is a, c, g, or t
<400> 8
   atggnctaca aggacgacga tgacaagggt gactacaagg acgacgatga caagggaggt      60
   ggtaccgagc tcggagaaaa cctgtatttt cagatcatcg gggacatgga ggccaagccc     120
   cactcccgcc cctacatggc ttatcttatg atctgggatc agaagtctct gaagaggtgc     180
   ggtggcttcc tgatacgaga cgacttcgtg ctgacagctg ctcactgttg gggaagctcc     240
   ataaatgtca ccttgggggc ccacaatatc aaagaacagg agccgaccca gcagtttatc     300
   cctgtgaaaa gacccatccc ccatccagcc tataatccta agaacttctc caacgacatc     360
   atgctactgc agctggagag aaaggccaag cggaccagag ctgtgcagcc cctcaggcta     420
   cctagcaaca aggcccaggt gaagccaggg cagacatgca gtgtggccgg ctgggggcag     480
   acggcccccc tgggaaaaca ctcacacaca ctacaagagg tgaagatgac agtgcaggaa     540
   gatcgaaagt gcgaatctga cttacgccat tattacgaca gtaccattga gttgtgcgtg     600
   ggggacccag agattaaaaa gacttccttt aagggggact ctggaggccc tcttgtgtgt     660
   aacaaggtgg cccagggcat tgtctcctat ggacgaaaca atggcatgcc tccacgagcc     720
   tgcaccaaag tctcaagctt tgtacactgg ataaagaaaa ccatgaaacg ctac           774
<210> 9
   <211> 22
   <212> PRT
   <213> artificial
<220>
   <223> FLAG-tag
<400> 9
   Met Asp Tyr Lys Asp Asp Asp Asp Lys Gly Asp Tyr Lys Asp Asp Asp
   1               5                   10                  15
   Asp Lys Gly Gly Gly Thr
               20

## Beschreibung

Die Erfindung betrifft ein Kombinationspräparat enthaltend ein selektives Zelltod-induzierendes binäres Enzymsystem zur Anwendung in der Therapie und/oder Behandlung von Krebs und Tumoren in Mensch und Tier, ein Verfahren und dessen Verwendung.

Krebs ist eine Klasse von Erkrankungen, die durch unkontrolliertes Zellwachstum und durch die Streuung entarteter Zellen im Körper gekennzeichnet ist und im Fall der Metastasierung schließlich zum Tod des Patienten führt. Die Behandlung von Tumoren und Krebserkrankungen hängt stark von der Art des auftretenden Tumors ab und erfolgt heute üblicherweise neben invasiven Eingriffen durch Anwendung einer Strahlen- oder Chemotherapie. Krebserkrankungen werden sowohl von externen Faktoren (Tabakrauchen, infektiöse Organismen oder Viren, Mutagene und ionisierende Strahlung) als auch internen Faktoren (genetische Prädisposition, Hormone, Faktoren des Immunsystems und spontan auftretende somatische Mutationen) ausgelöst. Weiterhin existieren zur Behandlung die Immuntherapie, Hormontherapie als auch die zielgerichtete Therapie. Die Vorteile einer Chemotherapie, Tumorzellen zu töten, liegt in ihrer Fähigkeit begründet, die Zellteilung durch Zerstören des Einwirkens auf die zelluläre DNA oder RNA zu unterbrechen. Sobald die Tumorzellen sich nicht mehr teilen können, sterben sie ab. Je schneller sich die Zellen teilen, umso höher ist die Wahrscheinlichkeit, dass sie durch das Chemotherapeutikum abgetötet werden können und ein Tumor durch Einleiten des Zelltods schrumpft. Folglich wirkt eine Chemotherapie am effizientesten auf solche Zellen, die sich schnell teilen. Die Chemotherapie ist jedoch nicht in der Lage, zwischen Krebs-/Tumorzellen und schnell wachsenden normalen Zellen des Körpers zu unterscheiden, so dass Nebenwirkungen wie Haarausfall, Müdigkeit, Schmerzen, Blutbildveränderungen und Übelkeit entstehen. Die Chemotherapie ist je nach Wirkmechanismus in fünf grobe Klassen zu unterteilen: Alkylierende Reagenzien, pflanzliche Alkaloide, Antitumor-Antibiotika und Antimetaboliten.

Im Rahmen der sogenannten zielgerichteten Therapien werden die Erkenntnisse aus den Unterschieden von Krebszellen gegenüber gesundnormalen Zellen genutzt. Die zielgerichtete Therapie soll die Krebszellen eliminieren, wobei spezifische Merkmale dieser Krebszellen genutzt werden, so dass keine Schädigung von normal gesunden Zellen erfolgt. Solche zielgerichtete Therapien umfassen als Wirkstoffe insbesondere monoklonale Antikörper, die die Krebszellen spezifisch erkennen und binden sowie Anti-Angiogenese Wirkstoffe, die das Wachstum der Blutgefäße zur Versorgung des Tumors spezifisch hemmen. Im Rahmen der zielgerichteten Therapie werden zumeist kleine organische Moleküle verwendet, die die Membran der Krebszelle durchdringen können und den zellulären Stoffwechsel blockieren und insbesondere eine Apoptose auslösen, die die Zellen abtöten. Es ist eine Vielzahl von Wirkstoffen beschrieben, die auf intrazelluläre Signalwege zielen, um eine solche Apoptose auszulösen. Andere Wirkstoffe erkennen und binden tumorspezifische Rezeptoren auf der Zelloberfläche.

Diese Therapien stellen jedoch für das Immunsystem eine außerordentliche Belastung dar und können in manchen Fällen lediglich eingeschränkt eingesetzt werden. Zudem erfordern diese Therapieformen zumeist lange Pausen zwischen den einzelnen Behandlungen zur Regeneration des Immunsystems. In den letzten Jahren haben sich daher neben diesen klassischen Maßnahmen insbesondere gentherapeutische Ansätze oder die genetische Vakzinierung zur Behandlung oder zur Unterstützung dieser Therapien als vielversprechend herausgestellt.

Die Gentherapie und die genetische Vakzinierung sind molekularmedizinische Verfahren, deren Anwendung allgemein in der Therapie und Prävention von Erkrankungen erhebliche Auswirkungen auf die medizinische Praxis haben. Beide Verfahren beruhen auf der Einbringung von Nukleinsäuren oder Peptiden in Zellen bzw. in Gewebe des Patienten sowie auf der anschließenden Verarbeitung der durch die eingebrachten Nukleinsäuren kodierten Information durch die Zellen bzw. das Gewebe, d.h. der Expression der erwünschten Polypeptide.

Die übliche Vorgehensweise bestehender Verfahren der Gentherapie und der genetischen Vakzinierung ist die Verwendung von DNA, um die benötigte genetische Information in die Zelle einzuschleusen. In diesem Zusammenhang sind verschiedene Verfahren zur Einbringung von DNA in Zellen, solche wie Calciumphosphat-Transfektion, Polypren-Transfektion, Protoplasten-Fusion, Elektroporation, Mikroinjektion und Lipofektion, beschrieben.

Ein weiteres Verfahren, das insbesondere bei genetischen Vakzinierungsverfahren vorgeschlagen wurde, ist die Verwendung von DNA-Viren als DNA-Vehikel. Derartige Viren haben den Vorteil, dass aufgrund ihrer infektiösen Eigenschaften eine sehr hohe Transfektionsrate zu erzielen ist.

Proteasen sind spezielle Proteine mit peptidolytischen und esterolytischen Eigenschaften und können in katalytischer Weise andere Substanzen und Proteine (Substrate) irreversibel verändern und umwandeln. Entsprechend der funktionell relevanten Molekülreste des katalytisch aktiven Zentrums werden diese Proteasen in vier verschiedene Hauptklassen eingeteilt: Serin-abhängige Proteasen, Cysteinproteasen, Aspartasen und Metalloproteasen. Proteasen vom Serin-Typus fallen in zwei große Familien, in die Familie der eigentlichen Serinproteasen und in die Subtilisin-Familie. Zu den bekanntesten Vertretern der Serinproteasen zählen die Verdauungsenzyme des Gastrointestinaltraktes, das Trypsin, Chymotrypsin und die Pankreas-Elastase, die bakteriziden und Matrix-abbauenden Enzyme der neutrophilen Granulozyten, Leukozyten-Elastase und Cathepsin G, die Kallikreine der Speicheldrüsen und die Serinproteasen des Gerinnungs- und Immunabwehrsystems. Serinproteasen in sekretorischen Granula von Mastzellen, Lymphozyten, Phagozyten oder natürlichen Killerzellen und die Serinproteasen des Komplementsystems spielen eine wichtige Rolle bei der Immunabwehr von Viren, Parasiten, Bakterien und Tumorzellen und bei Autoimmunprozessen. Serinproteasen sind auf unterschiedliche Substrate spezialisiert und können nach Asparaginsäure-Resten (Granzym B, Induktion der DNA- Fragmentierung in lysierten Zielzellen), Arginin- und Lysin-Resten (Trypsin, Granzym A und Granzym K), Methionin-Resten (Granzym M, "Met-ase") oder nach hydrophoben Aminosäuren (Elastase, Proteinase 3, Pankreas-Elastase, Chymotrypsin) eine Peptidbindung hydrolysieren. Eine Reihe von Lymphozyten-spezifischen Serinproteasen (Granzyme genannt) werden bei der Zielzell-Lyse sezerniert und sind direkt und indirekt nach Aufnahme in das Zytosol der Zielzelle am Vorgang der Zielzell-Zerstörung durch aktivierte Killerzellen beteiligt.

Die Apoptose einer Zelle kann durch verschiedene proapoptotische Mechanismen und Proteine induziert werden.

Gemeinsam ist diesen Mechanismen und Proteinen, dass sie eine proteolytische zelluläre Kaskadenreihe von Cysteinyl-Proteasen, genannt Caspasen, aktivieren. Die initial aktivierten Caspasen wie beispielsweise Caspase 8 und Caspase 9 aktivieren hierbei die Effektor-Kaskaden wie beispielsweise die Caspasen 3 und 6. Diese wiederum spalten eine Reihe von zellulären Substraten und bewirken hierdurch die Apoptose der betroffenen Zelle.

Im Rahmen dieser Erfindung kann der Begriff "programmierter Zelltod" synonym für "Apoptose" verwendet werden. Im Sinne dieser Erfindung ist ein "induzierter Zelltod", ein solcher, wobei mittels eines Wirkstoffes eine "Apoptose" bzw. "programmierter Zelltod" ausgelöst wird, vorzugsweise mittels einer Serinprotease.

Es ist jedoch bekannt, dass Serinproteasen zur Tumorbehandlung eingesetzt werden können.

Es gibt eine Reihe von Proteasen, die für ihre enzymatische Aktivität in ihren Substratproteinen eine spezifische Erkennungssequenz benötigen. Einige Beispiele dafür sind in der folgenden Tabelle aufgeführt. P1 bezeichnet die Position der Aminosäure, nach der geschnitten wird, P4, P3 und P2 sind die Positionen N-terminal vor der Schnittstelle P1. Die Bezeichnung P1' und P2' sind die Positionen C-terminal im Anschluss an P1. Das bedeutet, dass die Proteasen die Polypeptidkette zwischen P1 und P1' spalten.

| **Tabelle 1** | | | | | | |
|---|---|---|---|---|---|---|
| Protease | Schnittstelle | | | | | |
| | P4 | P3 | P2 | P1 | P1' | P2' |
| Caspase 1 | F, W, Y oder L | - | H, A oder T | D | nicht P, E, D, Q, K oder R | - |
| Caspase 2 | D | V | A | D | nicht P, E, D, Q, K oder R | - |
| Caspase 3 | D | M | Q | D | nicht P, E, D, Q, K oder R | - |
| Caspase 4 | L | E | V | D | nicht P, E, D, Q, K oder R | - |
| Caspase 5 | L oder W | E | H | D | - | - |
| Caspase 6 | V | E | H oder I | D | nicht P, E, D, Q, K oder R | - |
| Caspase 7 | D | E | V | D | nicht P, E, D, Q, K oder R | - |
| Caspase 8 | I oder L | E | T | D | nicht P, E, D, Q, K oder R | - |
| Caspase 9 | L | E | H | D | - | - |
| Caspase 10 | I | E | A | D | - | - |
| Clostripain (Clostridiopeptidase B) | - | - | - | R | - | |
| Enterokinase | D oder N | D oder N | D oder N | K | - | - |
| Faktor Xa | A, F, G, I, L, T, V oder M | D oder E | G | R | - | - |
| Granzyme B | I | E | P | D | - | - |
| Staphylococcus Peptidase I (V8 Protease) | - | - | nicht E | E | - | - |
| Thrombin | - | - | G | R | G | - |
| | A, F, G, I, L, T, V oder M | A, F, G, I, L, T, V, W or A | P | R | nicht D, E | nicht D, E |

### Aminosäuren im Einbuchstabencode

Eine besonders wirksame und spezifische Serinprotease (siehe Tabelle 1) ist Granzyme B, wie in SEQ ID No. 1 dargestellt.

Granzyme B (GzmB) stellt einen der Hauptbestandteile der Granula von cytotoxischen T-Zellen (kurz: CTL, veraltet: T-Killerzellen) dar. CTL sind in der Lage, aktives GzmB auf Zielzellen wie virusinfizierte Zellen oder Tumorzellen zu übertragen, so dass eine Apoptose ausgelöst wird. Die Erkennung von Zielzellen durch CTL erfordert jedoch die Aufnahme und Prozessierung von Antigenen durch dendritische Zellen (DC), ihre Präsentation auf MHC-Molekülen, sowie die Migration der entsprechenden DC zu den lokalen Lymphknoten, so dass antigenspezifische T-Zellen aktiviert werden können.

GzmB weist als Serinprotease die hochkonservierte N- terminale Konsensus-Sequenz Ile-(Ile/Val)-Gly-Gly auf, welche für die Erlangung der aktiven Konformation unerlässlich ist. Zur Überführung in die aktive Form (nachstehend: "aktives GzmB") ist es erforderlich, die N-terminale Konsensus-Sequenz Ile-(Ile/Val)-Gly-Gly bereitzustellen, wie in SEQ ID No. 2 gegeben.

Ausgehend von diesem Stand der Technik hat sich der Erfinder die Aufgabe gestellt, mittels eines Wirkstoffes einen induzierten Zelltod einer Krebs- oder Tumorzelle herbeizuführen.

Überraschender Weise können Tumorzellen mittels eines Zelltodinduzierenden binären Enzymsystem absterben und zwar bestehend aus einer Kombination enthaltend eine inaktive Form von Granzyme B umfassend SEQ ID No. 2 oder eine kodierende Nukleinsäure davon und tobacco etch virus protease (kurz: TEV) (SEQ ID No. 4 oder SEQ ID No. 5) oder eine kodierende Nukleinsäure davon.

Erfindungsgemäß erkennt TEV die recognition site (Erkennungssequenz) ENLYFQ (SEQ ID No. 6) oder ENLYFQG (SEQ ID No. 7) in der inaktiven Form, welche mit dem N-Terminus der SEQ ID No. 2 verknüpft (ligiert) ist. Erfindungsgemäß bevorzugt ist jedoch ENLYFQ (SEQ ID No. 6) in Ligation mit dem N-Terminus der SEQ ID No. 2, welche überraschender Weise zu einer verbesserten Spaltung führt.

Daher wird die Aufgabe durch die aufgestellten Patentansprüche vollumfänglich gelöst.

Sobald die inaktive Form von Granzyme B und TEV in einer Tumorzelle gemeinsam oder getrennt voneinander eingebracht und ggfs. exprimiert sind, setzt TEV die aktive Form von Granzyme B gemäß SEQ ID No. 2 frei und folglich erfolgt ein induzierter Zelltod im Wege der Apoptose oder einem programmierten Zelltod.

Besonders vorteilhaft ist die Auswahl der verwendeten Serinprotease und das Mittel zur Demaskierung von einer inaktiven Form in eine aktive Form von Granzyme B, nämlich mittels TEV. Sobald in einer Tumorzelle diese beiden Polypeptide vorliegen, verläuft die Demaskierung vollkommen spezifisch und effizient. Besonders vorteilhaft ist hierbei, dass weder PräGranzyme B noch TEV im Menschen oder Säugetier vorkommen.

Zu TEV wird auf das Dokument Kapust et al, The P1'specificity of tobacco etch virus protease, Biochemical and Biophysical Research Communications, 294 (2002) 949-955 verwiesen.

Daher betrifft die Erfindung ein Arzneimittel oder Kombinationspräparat zur Verwendung in der Behandlung und / oder Prophylaxe von Krebs- oder Tumorerkrankungen enthaltend eine inaktive Form von Granzyme B in einem Fusionsprotein umfassend SEQ ID No. 2 oder eine kodierende Nukleinsäure davon oder jeweils eine funktionelle Variante davon und TEV (SEQ ID No. 4 oder SEQ ID No. 5) oder eine kodierende Nukleinsäure davon oder jeweils eine funktionelle Variante davon, wobei die SEQ ID No. 2 mittels Spaltung durch TEV (SEQ ID No. 4 oder SEQ ID No. 5) erhalten oder freigesetzt wird. TEV erkennt die recognition site (Erkennungssequenz) ENLYFQ (SEQ ID No. 6) oder ENLYFQG (SEQ ID No. 7) in der inaktiven Form von Granzyme B.

In einer bevorzugten Ausführungsform der Erfindung ist die inaktive Form von Granzyme B ein PräGranzyme B (SEQ ID No. 3) oder eine kodierende Nukleinsäure davon (Zhinan Xia et al, Expression and Purfication of Enzymatically Active Recombinant Granzyme B in a Baculovirus System, Biochemical and Biophysical Research Communications, 243 (1998) 384-389). Eine solche kodierende Nukleinsäure ist z.B. SEQ ID No. 8.

In einer weiteren bevorzugten Ausführungsform der Erfindung ist die inaktive Form von Granzyme B ein Fusionsprotein umfassend SEQ ID No. 2 oder eine kodierende Nukleinsäure davon, wobei die SEQ ID No. 2 mittels Spaltung durch TEV (z.B. SEQ ID No. 4 oder SEQ ID No. 5) an ENLYFQ (SEQ ID No. 6) oder ENLYFQG (SEQ ID No. 7) erhalten oder freigesetzt wird.

Daher betrifft die Erfindung eine inaktive Form von Granzyme B und zwar ein Fusionsprotein umfassend SEQ ID No. 2 oder eine kodierende Nukleinsäure davon, wobei SEQ ID No. 2 mittels Spaltung durch TEV (z.B. SEQ ID No. 4 oder SEQ ID No. 5) an der Erkennungssequenz ENLYFQ (SEQ ID No. 6) oder ENLYFQG (SEQ ID No. 7) freigesetzt wird.

Daher betrifft die Erfindung eine inaktive Form von Granzyme B und zwar ein Fusionsprotein umfassend SEQ ID No. 2 und ENLYFQ (SEQ ID No. 6) oder ENLYFQG (SEQ ID No. 7) oder eine kodierende Nukleinsäure davon, und ggfs. weitere Sequenzen, wobei ENLYFQ (SEQ ID No. 6) oder ENLYFQG (SEQ ID No. 7) mit dem N-Terminus von SEQ ID. No. 2 verknüpft ist. Eine entsprechende Ausführungsform ist in SEQ ID No. 3 gegeben, und zwar vom Typ: FLAG-Tag-ENLYFQ-SEQ ID No.2. Weitere beliebige Fusionsproteine können entsprechend bereitgestellt werden (z.B. mittels HIS-tag, u.a.), wobei die beispielhafte FLAG-Tag durch ein beliebiges Peptid, beispielsweise 50 bis 100 Aminosäuren, ersetzt werden kann.

Der Fachmann ist in der Lage geeignete Fusionsproteine herzustellen und zu designen (Ausubel et al. (ed), (1989). Preparartion of Genomic DNA from Mammalian Tissue. In: Short Protocols in Molecular Biology: A Compendium of Methods from CURRENT PROTOCOLS IN MOLECULAR BIOLOGY. John Wiley & Sons).

Die erfindungsgemäßen Kombinationspräparate und Arzneimittel können mit geeigneten Hilfs- und Zusatzstoffen versetzt werden. Als geeignete Zusatz- und/oder Hilfsstoffe eignen sich z. B. eine physiologische Kochsalzlösung, Stabilisatoren, Proteinaseinhibitoren, Nukleaseinhibitoren etc.

Daher betrifft die Erfindung ebenfalls ein vorstehendes Kombinationspräparat oder Arzneimittel zur Anwendung oder Verwendung in der Behandlung und / oder Prophylaxe von Krebs- oder Tumorerkrankungen an Mensch und Tier, insbesondere Säugetier.

In einer weiteren bevorzugten Ausführungsform werden die erfindungsgemäßen Kombinationspräparate oder Arzneimittel mittels eines gentherapeutischen Verfahrens verabreicht.

Gentherapeutische Verfahren lassen sich z.B. dadurch erhalten, dass die erfindungsgemäßen Nukleinsäuren mit Liposomen komplexiert werden. Hierzu eignen sich Lipidmischungen wie bei Felgner, P.L. et al. (1987) Proc. Natl. Acad. Sci, USA 84, 7413; Behr, J.P. et al. (1989) Proc.Natl. Acad. Sci. USA 86, 6982; Felgner, J.H. et al. (1994) J. Biol. Chem. 269, 2550 oder Gao, X. & Huang, L. (1991) Biochim. Biophys. Acta 1189, 195 beschrieben. Bei der Herstellung der Liposomen wird die DNA ionisch auf der Oberfläche der Liposomen gebunden, und zwar in einem solchen Verhältnis, daß eine positive Nettoladung verbleibt und die DNA vollständig von den Liposomen komplexiert wird. Sterisch stabilisierte Liposome, die eine Hülle aus Polyethylenglykol (PEG) tragen, zeigen eine deutlich verringerte Aufnahme über das mononukleare Phagozytensystem (MPS), außerdem stark verlängerte Blutzirkulationszeiten, reduzierte Aggregation der PEGylierten Vesikel sowie eine verbesserte Stabilität der liposomalen Formulierungen. Analog zu PEG, zeigen lineares und hyperverzweigtes Polyglycerol (*l*PG und *hbPG*) exzellente Biokompatibilität, können aber durch die zusätzlichen Funktionalitäten weiter derivatisiert werden. Neuartige Lipide basierend auf hyperverzweigtem Polyglycerol, linearhyperverzweigtem PEG-*hb*PG-Blockcopolymeren und statistischen PEG-PG-Copolymeren wurden über kombinierte anionische Polymerisations von verschiedenen Epoxidmonomeren unter Verwendung lipophiler Initiatoren wie Cholesterin oder 1,2-Bis-n-alkylglycerylether hergestellt. Die neuen amphiphilen Strukturen wurden erfolgreich in liposomale Membranen mit 1,2-Dioleoyl-sn-glycero-3-phosphocholin (DOPC) als Colipid eingebracht.

Daher betrifft die Erfindung ebenfalls ein gentherapeutisches Verfahren wobei mittels eines Vehikels ein Einbringen in eine Zielzelle, vorzugsweise Tumorzelle, erfolgt.

In einer weiteren Ausführungsform kann dieses Vehikel ausgewählt sein aus der Gruppe Liposomen, Nano- oder Mikropartikel, Viren, Lipoplexe u.a. (Gene delivery by lipoplexes and polyplexes. Tros de Ilarduya C, Sun Y, Düzgüne 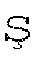 N. Eur J Pharm Sci. 2010 Jun 14; 40(3):159-70. doi: 10.1016/j.ejps.2010.03.019. Epub 2010 Mar 30; Efficient gene delivery by EGF-lipoplexes in vitro and in vivo, Buñuales M, Düzgünes N, Zalba S, Garrido MJ, de Ilarduya CT. Nanomedicine (Lond). 2011 Jan;6(1):89-98. doi: 10.2217/nnm.10.100; Genetic nanomedicine: gene delivery by targeted lipoplexes, Düzgüne 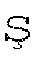 N, de Ilarduya CT. Methods Enzymol. 2012;509:355-67. doi: 10.1016/B978-0-12-391858-1.00018-6).

In einer besonders bevorzugten Ausführungsform weisen die erfindungsgemäßen Vehikel auf der Oberfläche Liganden auf, die Tumormarker erkennen. Solche Liganden können beispielsweise polyklonale oder monoklonale Antikörper oder kovalente Binder, Aptamere sein, die in der Lage sind an Tumormarker zu binden.

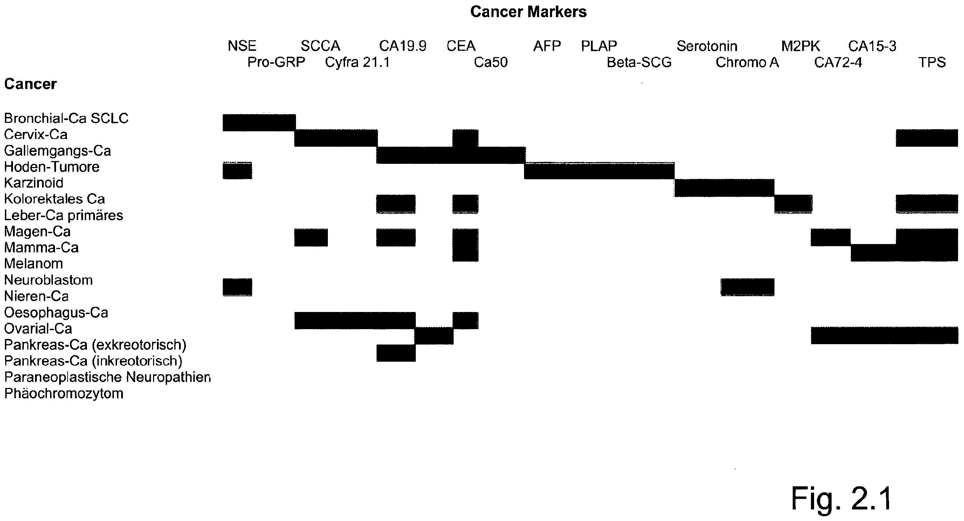

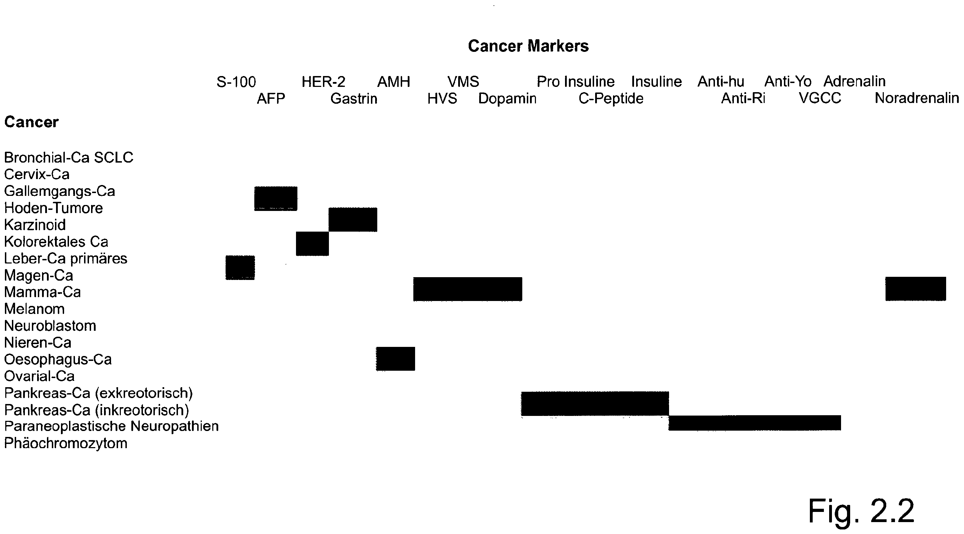

Solche präsentierende Tumormarker können nicht abschließend sein:
Carcioembryonales Antigen (CEA), Alpha Ferroprotein (AFP), Carbohydrate-Antigen 19/9 (CA 19-9), Cancer-Antigen 72/4 (CA 72-4), Cancer-Antigen 125, Cancer-Antigen 15/3 (CA 15-3), Neuronenspezifische Enolase (NSE), Squamous cell carcinoma antigen (SCC), Cytokeratin-Fragment (CYFRA), Humanes Choriongonadotropin (HCG), Prostataspezifisches Antigen (PSA), Humanes Thyreoglobulin (HTG), Mucin-like cancer associated antigen (MCA) u.a. Geeignete Tumormarker sind beispielsweise in Figur 2 in Abhängigkeit zur Krebserkrankung (Cancer) gezeigt.

Daher betrifft die Beschreibung ebenfalls ein Verfahren zum Einbringen eines erfindungsgemäßen Arzneimittels oder Kombinationspräparates, wobei eine inaktive Form von Granzyme B umfassend eine Nukleinsäure kodierend für SEQ ID No. 2 und eine Nukleinsäure kodierend für tobacco etch virus protease (z.B. SEQ ID No. 4 oder SEQ ID No. 5),
insbesondere eine inaktive Form von Granzyme B umfassend eine Nukleinsäure kodierend für ein Fusionsprotein umfassend SEQ ID No. 2 und ENLYFQ (SEQ ID No. 6) oder ENLYFQG (SEQ ID No. 7) und eine Nukleinsäure kodierend für tobacco etch virus protease,
i.) gemeinsam oder getrennt voneinander in mindestens einen Vehikel eingebracht werden,
ii.) in eine Tumorzelle eingebracht und exprimiert werden,
iii.) eine aktive Form des Granzyme B erhalten wird und ein induzierter Zelltod der Tumorzelle erfolgt.

Das Verfahren kann durch weitere vorgenannte Ausführungsformen entsprechend adaptiert werden. Die erfindungsgemäßen Arzneimittel, Kombinationspräparate, insbesondere deren Vehikel, können vorzugsweise lokal an Mensch und Tier verabreicht werden, z.B. subkutan verabreicht / appliziert werden. Selbstverständlich sind sämtliche Applikationen in der Tumorbehandlung erfindungsgemäß umfasst.

Unter dem Begriff "funktionelle Variante" im Sinne der vorliegenden Erfindung versteht man Polypeptide oder Nukleinsäuren, die funktionell mit dem erfindungsgemäßen Peptid verwandt sind. Unter Varianten versteht man ebenfalls allelische Varianten oder Polypeptide und Nukleinsäuren, die von anderen Organismen, Zellen bzw. Gewebe abstammen.

Im weiteren Sinne versteht man darunter auch Polypeptide oder Nukleinsäuren, die eine Sequenzhomologie, insbesondere eine Sequenzidentität von ca. 70%, vorzugsweise von ca. 80%, insbesondere von ca. 90%, vor allem von ca. 95% zu den ausgewiesenen SEQ ID haben.

Ferner zählen hierzu auch Deletion des Polypeptids im Bereich von ca. 1-50, vorzugsweise von ca. 1-30, insbesondere von ca. 1-15, vor allem von ca. 1-6 Aminosäuren. Beispielsweise kann die erste Aminosäure Methionin fehlen, ohne dass die Funktion des Polypeptids wesentlich verändert wird.

Daneben zählen hierzu auch Fusionsproteine, die die oben beschriebenen erfindungsgemäßen Polypeptide enthalten, wobei die Fusionsproteine selbst bereits die Funktion der jeweiligen SEQ ID haben oder erst nach Abspaltung des Fusionsanteils die spezifische Funktion bekommen können. Vor allem zählen hierzu Fusionsproteine mit einem Anteil von insbesondere nicht-humanen Sequenzen von ca. 1-50, vorzugsweise ca. 1-30 Aminosäuren. Beispiele von nicht-humanen Peptidsequenzen sind prokaryotische Peptidsequenzen, z. B. aus der Galactosidase von E. coli oder ein sogenannter Histidin-Tag, z. B. ein Met-Ala-His6-Tag. Ein Fusionsprotein mit einem sogenannten Histidin-Tag eignet sich besonders vorteilhaft zur Reinigung des exprimierten Proteins über Metallionen-haltige Säulen, beispielsweise über eine Ni²⁺-NTA-Säule. "NTA" steht für den Chelator "nitrilotriacetic acid" (Qiagen GmbH, Hilden). Insbesondere die genannten Teile des Polypeptids können auch mit Hilfe der klassischen Peptidsynthese (Merrifield-Technik) synthetisiert werden. Sie eignen sich insbesondere zur Gewinnung von Antiseren, mit deren Hilfe geeignete Genexpressionsbanken durchsucht werden können, umso zu weiteren funktionellen Varianten der erfindungsgemäßen Polypeptide zu gelangen.

In der Beschreibung ist die jeweils vorgenannte erfindungsgemäße Nukleinsäure eine DNA, cDNA oder RNA, vorzugsweise eine doppelsträngige DNA, jedoch ist ebenfalls eine PNA o.ä. denkbar.

Die erfindungsgemäßen Nukleinsäuren können ebenfalls mittels (Expressions)Vektoren in die Tumorzelle eingebracht werden. Beispielsweise mittels dem Vektor pcDNA 3.1 (Invitrogen) mit einem konstitutiven CMV Promoter u.a.

Im Sinne dieser Erfindung sind die Begriffe Tumor, Krebs, Krebszellen, Tumorzellen synonym zu lesen und umfasst jeden benignen oder malignen Tumor, insbesondere Geschwulst mit örtlich umschriebener Zunahme des Gewebevolumens, umfassend jede lokalisierte Anschwellung durch Ödem, akute und chronische Entzündung, aneurysmatische Erweiterung (pulsierender T.) u. a., auch entzündlich bedingte Organschwellung (z. B. als sog. Milztumor) als auch gewebliche Neubildung (Gewächs, Blastom, Neoplasie) in Form eines spontanen, verschiedengradig enthemmten, autonomen u. irreversiblen Überschusswachstums von körpereigenem Gewebe, das in der Regel mit unterschiedlich ausgeprägtem Verlust spezif. Zell- u. Gewebefunktionen verbunden ist (siehe Pschyrembel, (261. Auflage) 2007, de Gruyter, Berlin).

### Beispiele und Figuren:

Diese Beispiele dienen ausschließlich zur Erläuterung der Erfindung, ohne die Erfindung auf diese Beispiele zu beschränken.

### Beispiel:

### Beispiel 1

### Herstellung des pSTdna 1023-Plasmid

Die Tabacco-Etch-Virus-Protease-Sequenz wurde in den Vektor pcDNA™3.1 (Firma Invitrogen) einligiert. Zusätzlich wurde die FLAG-Sequenz (MDYKDDDDKGDYKDDDDKGGGT) zweifach in die n-terminale Region einkloniert.

### Herstellung von pSTdna 1024 ("GrzB WT")

Ein zusätzliches Plasmid basierend auf dem Vektor pcDNA™3.1 wurde mit der folgenden Reihenfolge kloniert und zwar zweimal FLAG-Sequenz (MDYKDDDDKGDYKDDDDKGGGT) (SEQ ID No. 9) in Verbindung mit TEV-Recognition-Sequence (SEQ ID No. 6) und Granzyme B AA21-240.

### Herstellung des Plasmid pSTdna 1025

Die Aminosäure 203-Serin wurde durch 203-Alanin im Plasmid pSTdna 1024 ersetzt. Diese Mutation inhibiert die Granzyme-B-Aktivität spezifisch ("GrzB mut").

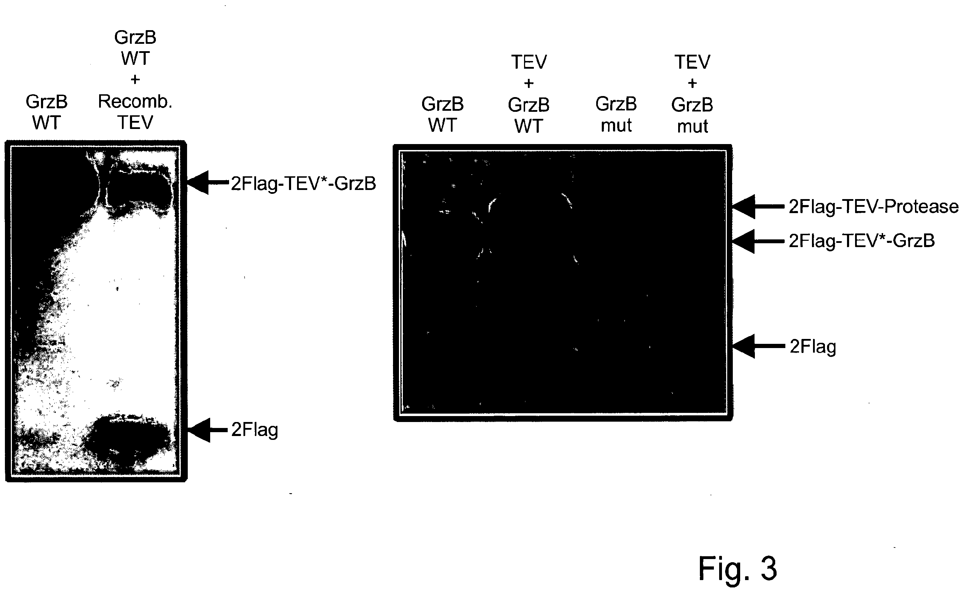

Die Spaltungsergebnisse mit TEV sind in Figur 3 dargestellt. Sofern die Recognition site (SEQ ID No. 6) statt Q ein G aufweist, findet keine Spaltung durch TEV statt (nicht gezeigt).

### Beispiel 2

### Protokoll

1. Die HeLa-Zellen wurden in DMEM-Medium bei 37C und 5% CO2 bis zu 75% Konfluenz kultiviert.
2. Das Medium wurde vorsichtig abgesaugt und die Zellen wurden mit PBS gewaschen.
3. Die Zellen wurden mit Hilfe von Trypsin-EDTA (200ml/L) 5 Min. verdaut, so dass die Zellen von der Petri-Platte gelöst werden. Die Verdauung wurde bei Zufuhr von DMEM gestoppt.
4. Die Zellen wurden 5 Min. bei 500g abzentrifugiert und zur Elektroporation in einem Medium (Lonza) mit einem 1µg Plasmid resuspendiert. Anschließend wurden die Zellen mit einem Amaxxa-Nucleofaktor-II-Elektroporator elektroporiert und in frischer DMEM resuspendiert.
5. Die Zellen wurden 4h bei 37°C und 5% CO₂ inkubiert.
6. A:1µg Plasmid wurde mit 100µl Opti-MEM in eine 1,5mL-Tube gemischt.
7. B:8µl Lipofectamine wurden in eine 1,5mL-Tube gemischt und 5 Min. bei Raumtemperatur inkubiert.
8. A und B wurden zusammen pipettiert und vorsichtig gemischt. Die Inkubationszeit beträgt 30 Min. bei Raumtemperatur.
9. Das Zell-Medium wurde vorsichtig abgesaugt und die Zellen wurden zweimal mit Opti-MEM gewaschen. Die Inkubation der Zellen erfolgt mit 3mL Opti-MEM bei 37°C und 5% CO₂.
10. Nach 30 Min. Inkubation der Mischung A und B aus Schritt 8 wurde AB zu den Opti-MEM pipettiert und vorsichtig gemischt.
11. Die Zellen wurden 5 Stunden bei 37°C und 5% CO₂ inkubiert.
12. Nach 5h wurde 5mL DMEM hinzugegeben.

### Beschreibung der Figuren:

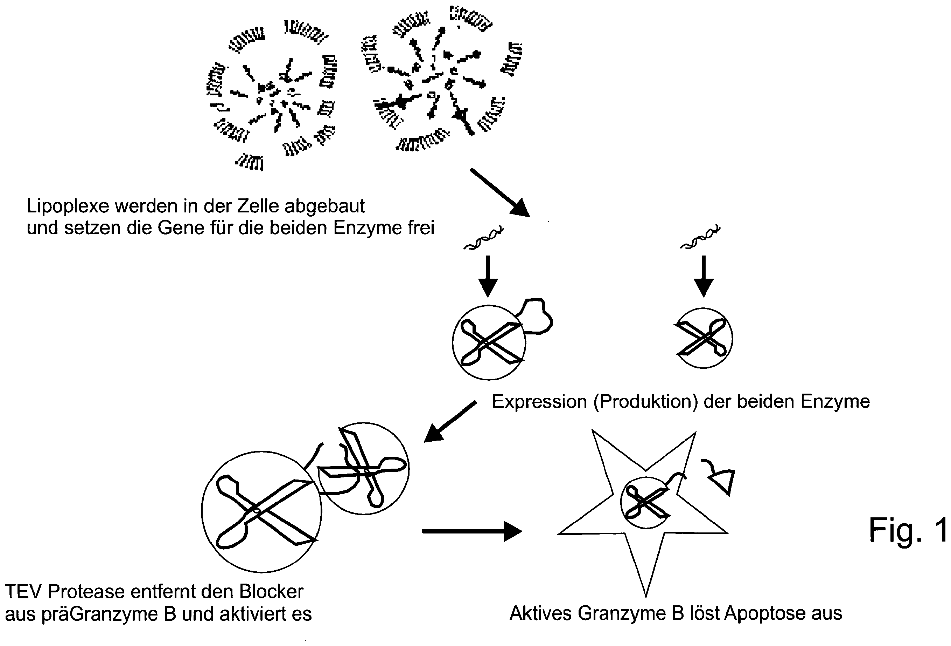

Figur 1 beschreibt die erfindungsgemäße Therapiestrategie zum induzierten Zelltod der Tumorzellen.
Figur 2 zeigt Tumormarker für bestimmte Krebserkrankungen.
Figur 3 zeigt TEV-Spaltungsergebnisse nach Beispiel 1.

## Patentansprüche

1. Arzneimittel zur Verwendung in der Behandlung und / oder Prophylaxe von Krebs- oder Tumorerkrankungen enthaltend eine inaktive Form von Granzyme B in einem Fusionsprotein umfassend SEQ ID No. 2 oder eine kodierende Nukleinsäure davon oder jeweils eine funktionelle Variante davon und tobacco etch virus protease (SEQ ID No. 4 oder SEQ ID No. 5) oder eine kodierende Nukleinsäure davon oder jeweils eine funktionelle Variante davon, **dadurch gekennzeichnet, dass** die SEQ ID No. 2 oder eine funktionelle Variante davon mittels Spaltung durch tobacco etch virus protease (SEQ 1D No. 4 oder SEQ ID No. 5) oder eine funktionelle Variante davon erhalten oder freigesetzt wird.

2. Arzneimittel zur Verwendung nach Anspruch 1, **dadurch gekennzeichnet, dass** die inaktive Form von Granzyme B ein PräGranzyme B (SEQ ID No. 3) oder eine kodierende Nukleinsäure davon oder jeweils eine funktionelle Variante davon ist.

3. Arzneimittel zur Verwendung nach Anspruch 1, **dadurch gekennzeichnet, dass** die inaktive Form von Granzyme B ein Fusionsprotein umfassend SEQ ID No. 2 ist oder eine kodierende Nukleinsäure davon oder jeweils eine funktionelle Variante davon ist, wobei ENLYFQ (SEQ ID No. 6) oder ENLYFQG (SEQ ID No. 7) mit dem N-Terminus der SEQ ID No. 2 ligiert ist, und SEQ ID No. 2 oder eine funktionelle Variante davon mittels Spaltung durch tobacco etch virus protease an ENLYFQ (SEQ ID No. 6) oder ENLYFQG (SEQ ID No. 7) erhalten oder freigesetzt wird.

4. Kombinationspräparat enthaltend ein Arzneimittel zur Verwendung in der Behandlung und / oder Prophylaxe von Krebs- oder Tumorerkrankungen gemäß einem der Ansprüche 1 bis 3, ggfs. Hilfs- und Zusatzstoffe.

5. Kombinationspräparat zur Verwendung in der Behandlung und / oder Prophylaxe von Krebs- oder Tumorerkrankungen gemäß Anspruch 4 an Mensch und Tier.

6. Kombinationspräparat oder Arzneimittel zur Verwendung in der Behandlung und / oder Prophylaxe von Krebs- oder Tumorerkrankungen gemäß einem der vorstehenden Ansprüche, **dadurch gekennzeichnet, dass** dieses mittels einem gentherapeutischen Verfahren verabreicht wird.

7. Kombinationspräparat oder Arzneimittel zur Verwendung in der Behandlung und / oder Prophylaxe von Krebs- oder Tumorerkrankungen gemäß einem der vorstehenden Ansprüche, **dadurch gekennzeichnet, dass** dieses gentherapeutische Verfahren mittels eines Vehikels erfolgt.

8. Kombinationspräparat oder Arzneimittel zur Verwendung in der Behandlung und / oder Prophylaxe von Krebs- oder Tumorerkrankungen gemäß einem der vorstehenden Ansprüche, **dadurch gekennzeichnet, dass** diese Vehikel aus cer Gruppe Liposomen, Nano- oder Mikropartikel, Viren, Lipoplexe ausgewählt sind.

9. Kombinationspräparat oder Arzneimittel zur Verwendung in der Behandlung und / oder Prophylaxe von Krebs- oder Tumorerkrankungen gemäß einem der vorstehenden Ansprüche, **dadurch gekennzeichnet, dass** diese Vehikel Liganden enthalten die Tumormarker erkennen.

10. Kombinationspräparat oder Arzneimittel zur Verwendung in der Behandlung und / oder Prophylaxe von Krebs- oder lumorerkrankungen gemäß einem der vorstehenden Ansprüche, **dadurch gekennzeichnet, dass** eine inaktive Form von Granzyme B umfassend eine Nukleinsäure kodierend für SEQ ID No. 2 oder eine funktionelle Variante davon und eine Nukleinsäure kodierend für tobacco etch virus protease oder eine funktionelle Variante davon,
i.) gemeinsam oder getrennt voneinander in mindestens ein Vehikel eingebracht werden,
ii.) in eine Tumorzelle eingebracht und exprimiert werden,
iii.) eine aktive Form des Granzyme B erhalten wird und ein induzierter Zelltod der Tumorzelle erfolgt.

## Claims

1. A drug for use in the treatment and/or prophylaxis of cancer or tumor diseases containing an inactive form of granzyme B in a fusion protein comprising SEQ ID no. 2 or a nucleic acid encoding it, or a functional variant of either, and tobacco etch virus protease (SEQ ID no. 4 or SEQ ID no. 5) or a nucleic acid encoding it or a functional variant of either, **characterized in that** the SEQ ID no. 2 or a functional variant of it is obtained or released by means of cleavage by tobacco etch virus protease (SEQ ID no. 4 or SEQ ID no. 5) or a functional variant of it.

2. The drug for use according to claim 1, **characterized in that** the inactive form of granzyme B is a pre-granzyme B (SEQ ID no. 3) or a nucleic acid encoding it, or a functional variant of either.

3. The drug for use according to claim 1, **characterized in that** the inactive form of granzyme B is a fusion protein comprising SEQ ID no. 2 or a nucleic acid encoding it, or a functional variant of either, wherein ENLYFQ (SEQ ID no. 6) or ENLYFQG (SEQ ID no. 7) is ligated with the N-terminus of SEQ ID no. 2 and SEQ ID no. 2 or a functional variant of it is obtained or released by means of cleavage by tobacco etch virus protease at ENLYFQ (SEQ ID no. 6) or ENLYFQG (SEQ ID no. 7).

4. A combination preparation containing a drug for use in the treatment and/or prophylaxis of cancer or tumor diseases according to one of claims 1 through 3, and possibly excipients and additives.

5. The combination preparation for use in the treatment and/or prophylaxis of cancer or tumor diseases according to claim 4 in humans and animals.

6. The combination preparation for use in the treatment and/or prophylaxis of cancer or tumor diseases according to one of the preceding claims, **characterized in that** it is administered by means of a gene therapy process.

7. The combination preparation for use in the treatment and/or prophylaxis of cancer or tumor diseases according to one of the preceding claims, **characterized in that** this gene therapy process is carried out by means of a vehicle.

8. The combination preparation for use in the treatment and/or prophylaxis of cancer or tumor diseases according to one of the preceding claims, **characterized in that** this vehicle is selected from the group of liposomes, nano- or microparticles, viruses, and lipoplexes.

9. The combination preparation for use in the treatment and/or prophylaxis of cancer or tumor diseases according to one of the preceding claims, **characterized in that** these vehicles contain ligands that recognize tumor markers.

10. The combination preparation for use in the treatment and/or prophylaxis of cancer or tumor diseases according to one of the preceding claims, **characterized in that** an inactive form of granzyme B comprising a nucleic acid encoding SEQ ID no. 2, or a functional variant of it, and a nucleic acid encoding tobacco etch virus protease or a functional variant of it,
i.) are introduced, together or separately from one another, in at least one vehicle,
ii.) into a tumor cell and expressed there,
iii.) producing an active form of granzyme B and inducing cell death in the tumor cell.

## Revendications

1. Produit pharmaceutique destiné à l'utilisation dans le traitement et / ou la prophylaxie de maladies cancéreuses ou tumorales, contenant une forme inactive de la granzyme B dans une protéine de fusion comprenant la SEQ ID N ° 2 ou un acide nucléique codant pour celle-ci ou respectivement une de ses variantes fonctionnelles, et de la protéase de virus de la gravure du tabac (SEQ ID N °4 ou SEQ ID N °5), ou un acide nucléique codant pour celle-ci, ou respectivement une de ses variantes fonctionnelles, **caractérisé en ce que** la SEQ ID N ° 2, ou une de ses variantes fonctionnelles, peut être obtenue ou libérée au moyen d'une scission par la protéase du virus de la gravure de tabac (SEQ ID N ° 4 ou SEQ ID N ° 5) ou une de ses variantes fonctionnelles.

2. Produit pharmaceutique destiné à l'utilisation selon la revendication 1, **caractérisé en ce que** la forme de la granzyme B est une pré-granzyme B (SEQ ID N °3) ou un acide nucléique codant pour celle-ci, ou respectivement une de ses variantes fonctionnelles.

3. Produit pharmaceutique destiné à l'utilisation selon la revendication 1, **caractérisé en ce que** la forme inactive de la granzyme B est une protéine de fusion comprenant la SEQ ID N °2 ou un acide nucléique codant pour celle-ci, ou respectivement une de ses variantes fonctionnelles, où de l'ENLYFQ (SEQ ID N °6) ou de l'ENLYFQG (SEQ ID N °7) sont ligués avec l'extrémité N-terminale de la SEQ ID N ° 2, et la SEQ ID N ° 2, ou une de ses variantes fonctionnelles, sont obtenues ou libérées en ENLYFQ (SEQ ID N °6) ou en ENLYFQG (SEQ ID N ° 7) au moyen d'une scission par la protéase de virus de la gravure de tabac.

4. Préparation combinée contenant un produit pharmaceutique destiné à l'utilisation dans le traitement et / ou la prophylaxie de maladies cancéreuses ou tumorales selon l'une des revendications 1 à 3, éventuellement, des produits auxiliaires ou additifs.

5. Préparation combinée destinée à l'utilisation dans le traitement et / ou la prophylaxie de maladies cancéreuses ou tumorales selon la revendication 4, sur l'humain et l'animal.

6. Préparation combinée ou produit pharmaceutique destiné(e) à l'utilisation dans le traitement et / ou la prophylaxie de maladies cancéreuses ou tumorales selon l'une des revendications précédentes, caractérisé(e) en ce que celle-ci ou celui-ci est administré(e) au moyen d'un procédé de thérapie génique.

7. Préparation combinée ou produit pharmaceutique destiné(e) à l'utilisation dans le traitement et / ou la prophylaxie de maladies cancéreuses ou tumorales selon l'une des revendications précédentes, caractérisé(e) en ce que ce procédé de thérapie génique a lieu au moyen d'un véhicule.

8. Préparation combinée ou produit pharmaceutique destiné(e) à l'utilisation dans le traitement et / ou la prophylaxie de maladies cancéreuses ou tumorales selon l'une des revendications précédentes, caractérisé(e) en ce que ces véhicules sont choisis dans le groupe à base de liposomes, de nanoparticules ou de microparticules, de virus, de lipoplexes.

9. Préparation combinée ou produit pharmaceutique destiné(e) à l'utilisation dans le traitement et / ou la prophylaxie de maladies cancéreuses ou tumorales selon l'une des revendications précédentes, **caractérisé en ce que** ces véhicules contiennent des ligands qui reconnaissent des marqueurs tumoraux.

10. Préparation combinée ou produit pharmaceutique destiné(e) à l'utilisation dans le traitement et / ou la prophylaxie de maladies cancéreuses ou tumorales selon l'une des revendications précédentes, caractérisé(e) en ce qu'une forme inactive de la granzyme B comprenant un acide nucléique codant pour la SEQ ID N °2, ou une de ses variantes fonctionnelles, et un acide nucléique codant pour la protéase de virus de gravure de tabac ou une de ses variantes fonctionnelles,
i.) sont incorporés ensemble ou séparés l'un de l'autre dans au moins un véhicule,
ii.) sont incorporés et exprimés dans une cellule tumorale,
iii.) une forme active de la granzyme B est obtenue et qu'il se produit une mort cellulaire induite de la cellule tumorale.
